# EUROPEAN PATENT SPECIFICATION

(11) **EP 2 173 885 B1**
(45) Date of publication and mention of the grant of the patent: **03.05.2017**
(21) Application number: 08779135.6
(22) Date of filing: 26.06.2008
(51) Int. Cl.: C12N 15/82, C07K 14/02

(54) **EXPRESSION CASSETTE, T-DNA MOLECULE, PLANT EXPRESSION VECTOR, TRANSGENIC PLANT CELL AS WELL AS THEIR USE IN THE MANUFACTURING OF A VACCINE**
EXPRESSIONSKASSETTE, T-DNA-MOLEKÜL, PFLANZENEXPRESSIONSVEKTOR, TRANSGENE PFLANZENZELLE SOWIE DEREN VERWENDUNG BEI DER HERSTELLUNG EINES IMPFSTOFFS
CASSETTE D'EXPRESSION, MOLÉCULE D'ADN-T, VECTEUR D'EXPRESSION DE PLANTE, CELLULE DE PLANTE TRANSGÉNIQUE AINSI QUE LEUR UTILISATION DANS LA FABRICATION D'UN VACCIN

(30) Priority: 28.06.2007 PL 38276907
(43) Date of publication of application: 14.04.2010
(73) Proprietor: Instytut Genetyki Roslin Polskiej Akademii Nauk, 60-479 Poznan (PL); Instytut Biotechnologii I Antybiotykow, 02-516 Warszawa (PL); Instytut Wlokien Naturalnych I Roslin Zielarskich, 60-630 Poznan (PL); Medana Pharma Terpol Group S.a., 98-200 Sieradz (PL); Centrum Badan DNA Sp. z o.o., 61-612 Poznan (PL)
(72) Inventor: WOJCIECHOWICZ, Jacek, 61-616 Poznan (PL); PNIEWSKI, Tomasz, PL-60-479 Poznan (PL); KAPUSTA, Jósef, PL-60-479 Poznan (PL); BOCIAG, Piotr, PL-60-479 Poznan (PL); KOSZTRZAK, Anna, PL-60-479 Poznan (PL); WOLKO, Bogdan, PL-60-479 Poznan (PL); PLUCIENNICZAK, Andrzej, PL-60-479 Poznan (PL); PLUCIENNICZAK, Grazyna, PL-60-479 Poznan (PL); WÓJCIK, Piotr, PL-61-707 Poznan (PL); OTTA, Halina, PL-61-707 Poznan (PL)
(74) Representative: Witek, Rafal
(86) International application number: PCT/PL2008/000046
(87) International publication number: WO 2009/002198

(56) References cited:
- EP-A- 1 093 820
- US-B1- 6 551 820
- KAPUSTA J ET AL: "A plant-derived edible vaccine against hepatitis B virus" FASEB JOURNAL, FED. OF AMERICAN SOC. FOR EXPERIMENTAL BIOLOGY, BETHESDA, MD, US, vol. 13, no. 13, 1 January 1999 (1999-01-01), pages 1796-1799, XP002219288 ISSN: 0892-6638 cited in the application
- UMABALLAVA MOHAPATRA ET AL: "Expression of the Bar Gene Confers Herbicide Resistance in Transgenic Lettuce" TRANSGENIC RESEARCH, KLUWER ACADEMIC PUBLISHERS-PLENUM PUBLISHERS, NE, vol. 8, no. 1, 1 February 1999 (1999-02-01), pages 33-44, XP019269532 ISSN: 1573-9368
- DATABASE EMBL [Online] 4 August 1994 (1994-08-04), "Human hepatitis B virus (ayw4) genes for surface protein S, core protein, protein X, DNA polymerase" XP002505988 retrieved from EBI accession no. EMBL:Z35716 Database accession no. Z35716
- KAPUSTA J ET AL: "Oral immunization of human with transgenic lettuce expressing hepatitis B surface antigen." ADVANCES IN EXPERIMENTAL MEDICINE AND BIOLOGY 2001, vol. 495, 2001, pages 299-303, XP009109297 ISSN: 0065-2598 cited in the application
- KUMAR G B SUNIL ET AL: "Production of hepatitis B surface antigen in recombinant plant systems: An update" BIOTECHNOLOGY PROGRESS, vol. 23, no. 3, May 2007 (2007-05), pages 532-539, XP009109296 ISSN: 8756-7938

## Description

The present invention relates to the production of an oral vaccine against viral hepatitis type B (Hep B) in the form of preserved plant material.

Although a recombinant vaccine against hepatitis B, meaning viral hepatitis type B, has been available for prophylaxis since the early 1980's, the global number of the chronically ill as a result of HBV infection has been growing annually. Whereas the number of HBV carriers was estimated at around 300 million persons at the end of the 80's and 350 million in the 90's, current data indicate some 400 million. It is estimated that close to 100 million of these patients may die due to diseases and other complications, for example cirrhosis or cancer of the liver. At the same time, the population of persons chronically ill with HepB comprises a huge reservoir for new infections. Approximate epidemiological data suggest that close to 1/3 of the global population has undergone HBV infection. And whereas HepB infection rates are rather low in developed nations, less than 0.5%, the index in developing nations and China is from 5 to 50% (Hollinger 1996, Young et al. 2001). HepB infection rates have been and continue to be serious epidemiological problems in developed nations as well, for example in Poland. Among virus-borne hepatic diseases, over 50% is caused by HBV (Walewska-Zielecka et al. 1996). It is also estimated that up to 20% of the population of industrialized nations test positive for HBV infection markers.

The number of the chronically ill and carriers of the virus is on the rise, despite the introduction of a recombinant vaccine some 20 years ago, based on the so-called small surface antigen of the virus, meaning S-HBs, produced in yeast cells (rHBsAg), which has facilitated large-scale vaccination. It has been found that in a number of vaccinated persons the virus successfully infects and undergoes replication. The infection occurs as a result of the appearance of mutated strains, characterized by amino-acid substitutions within epitope "a" of the small subunit of the S-HBs surface antigen. This epitope is representative and neutralizing for all subtypes of the virus described so far. It is supposed that these mutations occur due to the selective pressure caused by the antibodies produced during following the vaccination. New, mutant variants of epitope "a" are characterized by altered immunogenicity. Therefore, a sporadic lack of adequate immune protection against virus strains containing some mutations are observed, along with the development of chronic disease, despite a prior response at a level of ≥10 mIU/ml anti-HBs antibodies, which in most nations is thought to be sufficiently high to provide immune protection against viral infection (Cooreman et al. 2001, Huang et al. 2004).

For the above reasons, the design of alternative, easily available and highly effective vaccine against HepB was and remains commonly desirable.

The crux of producing an orally applied vaccine against HepB lays in the expression and efficient production of the highly immunogenic small subunit of the S-HBs surface antigen of HBV in a transgenic, edible plant. Research on this subject has been performed for a number of years by several groups (see the literature cited). To date, the transgenic plants produced or tissue/cell cultures were characterized in each case by antibiotic resistance, chiefly against kanamycin. At the same time, the S-HBsAg expression level oscillated in the range of 1-3 *µ*g/gram fresh weight (FW), and maximally amounted to 16 *µ*g/g FW. Only unprocessed, raw, plant material was used in oral vaccination studies in animals or volunteers.

The goal of the present invention is the production of an alternative oral vaccine against HepB produced using transgenic edible plants. In particular the goal of the present invention is the efficient expression of the S-HBs antigen in edible transgenic plants, i.e. lettuce, which facilitates the production of an oral vaccine, for example in the form of a suspension, syrup or granulate to be used on a wide scale, both as a primary vaccine or as a booster vaccine for persons immunized previously, in whom the anti-HBs antibody titre has decreased, or anti-HBs antibodies are undetectable.

Unexpectedly the above stated goals have been achieved in the present invention.

The subject of the present invention is an expression cassette containing a sequence encoding the small subunit of the HBV surface antigen (S-HBsAg) and regulatory sequences controlling its expression: the 35S RNA promoter of the cauliflower mosaic virus (CaMV) along with the terminator sequence of nopaline synthase (NOSt), wherein said expression cassette possesses the sequence represented as SEQ ID No. 1.

The next subject of the present invention is a T-DNA molecule containing flanking T-DNA sequences and two expression cassettes located between them:
- an expression cassette according to the invention defined above and
- an expression cassette composed of a sequence encoding herbicide resistance gene, preferentially *bar* gene, as well as regulatory sequences controlling its expression, preferentially the nopaline synthase promoter (PNOS) and the g7t transcription terminator.

The next subject of the present invention is an expression vector containing an expression cassette according to the present invention defined above, preferentially contained in a T-DNA molecule according to the present invention as defined above. In an example embodiment this is the binary vector pKHBSBAR.

The next subjects of the present invention are strains of *E. coli* as well as *Agrobacterium tumefaciens* containing an expression vector according to the present invention.

The next subject of the present invention is the use of a plant expression vector according to the present invention in the production of transgenic plants, preferentially lettuce.

The next subject of the present invention is a transgenic plant cell containing a plant expression vector according to the present invention, capable of expressing the small surface antigen protein of HBV, S-HBsAg, and possessing resistance to phosphinothricin herbicides. Preferably, it is a lettuce cell.

In particular, the embodiment of the present invention are transgenic and regenerated cells, as well as subsequent plant progeny generations of lettuce, characterized in that due to their transformation with a vector according to the present invention, they express the small surface subunit of HBV (S-HBsAg) as well as being resistant to phosphinothricin and derivative herbicides. Plant material derived from them may be used as an oral vaccine against HBV and, as a result, against HepB. Following dehydration via lyophilisation using a freeze-drying technique, the preparation retains the native structure of the antigenic protein at room temperature and immunogenicity for a period of at least 12 months and may be used in the production of an oral vaccine against HepB in the form of a suspension, syrup, granulate, tablets or capsules.

The next subject of the present invention is the use of a plant cell according to the present invention in the production of an oral vaccine against viral hepatitis type B. Preferentially, the plant cells used are in the form of plant biomass, particularly lyophilised plant material, preferentially lettuce.

Lyophilised plant material according to the present invention administered *per os* to experimental animals in the form of a suspension elicits an immune response in the mucous membranes characterised by the production of IgA anti-SHBs antibodies as well as a systemic response characterised by the production of IgA and IgG anti-SHBs antibodies. In particular, the lyophilised plant material according to the present invention administered *per os* to experimental animals previously vaccinated *per os* a single time elicited a boost of the mucous membrane immune response characterised by the production of IgA anti-SHBs antibodies as well as boosting the systemic response characterised by the production of IgA and IgG anti-SHBs antibodies.

Preferentially, the vaccine produced is in the form of: a suspension, syrup, granulate, tablet or capsule. Preferentially, the granulate, tablets as well as capsules formed from pulverised transgenic lettuce lyophilisate according to the present invention retain the native structure of antigenic proteins and their immunogenicity at room temperature for a period of at least 12 months.

In relation to results known from the state of the art, the present invention presents significant novelty and a fundamentally innovative approach to the production of an oral vaccine, encompassing the following elements:
- transgenic lettuce producing S-HBsAg at a level in excess of 20 µg S-HBsAg/g FW and up to 60 µg/g FW, without containing antibiotic resistance marker genes, but at the same time resistant to phosphinothricin herbicides such as Basta, and thus poses no risk of enhancing the antibiotic resistance of microflora resident in humans
- the initial production, from transgenic lettuce producing S-HBs, of a prototype vaccine, condensed as a lyophilisate in the form of: a suspension or syrup, as well as a granulate, tablets or capsules
- initial oral immunization using a suspension of the lyophilisate as well as a syrup, granulate, tablets or capsules.

### Detailed description of the present invention

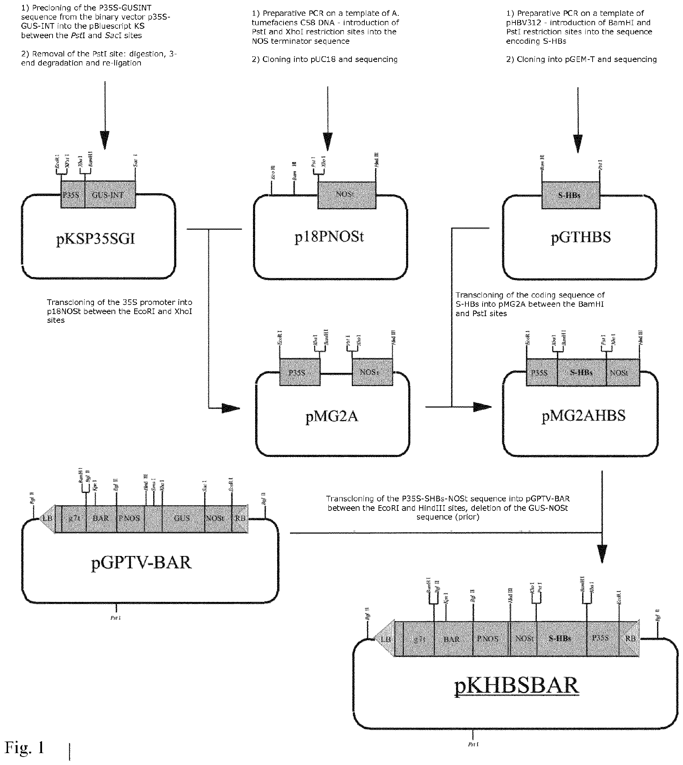

One of the preferential embodiments of the present invention is the pKHBSBAR vector [Fig. 1] for the production of plants resistant to phosphinothricin pesticides such as Basta and at the same time expressing the heterologous S-HBsAg protein.

An important characteristic of the vector is a T-DNA containing two expression cassettes, determining the expression of the immunogenic protein of the S subunit of the HBs surface antigen (subtype *ayw4, adw4*) of the hepatitis type b virus (HBV) as well as phosphinothricin acetyltransferase, bestowing resistance to phosphinothricin, which is an ingredient of a number of non-selective herbicides such as Basta. The first cassette contains a sequence encoding S-HBsAg under the control of the following regulatory sequences: the CaMV 35S RNA promoter with a single enhancer and the nopaline synthase transcription terminator (NOSt). The second cassette consists of a sequence encoding the *bar* gene under the control of the nopaline synthase promoter (PNOS) and the transcription terminator g7t. Another significant feature of the T-DNA construct is the occurrence of the above-mentioned coding and regulatory sequences strictly in one copy and in a particular orientation in relation to one another [Fig. 1]. A lack of sequence motif repeats eliminates recombinations within the introduced T-DNA as well as the balanced transcription of individual transgenes. The structural characteristics of the T-DNA thus act in concert to limit gene silencing, and by the same token greater T-DNA stability within the genome of transgenic plants, which is of particular importance in the case of lettuce whose genome is relatively variable and prone to rearrangements (McCabe et al. 1999). As a consequence, the structural characteristics of the T-DNA promote the stable expression of S-HBs transgenes as well as of *bar* in plant cells.

The next aspect of the present invention is a method of transforming lettuce using an *Agrobacterium tumefaciens* strain containing a vector for transformation as well as the regeneration of lettuce, *Lactuca sativa* L.

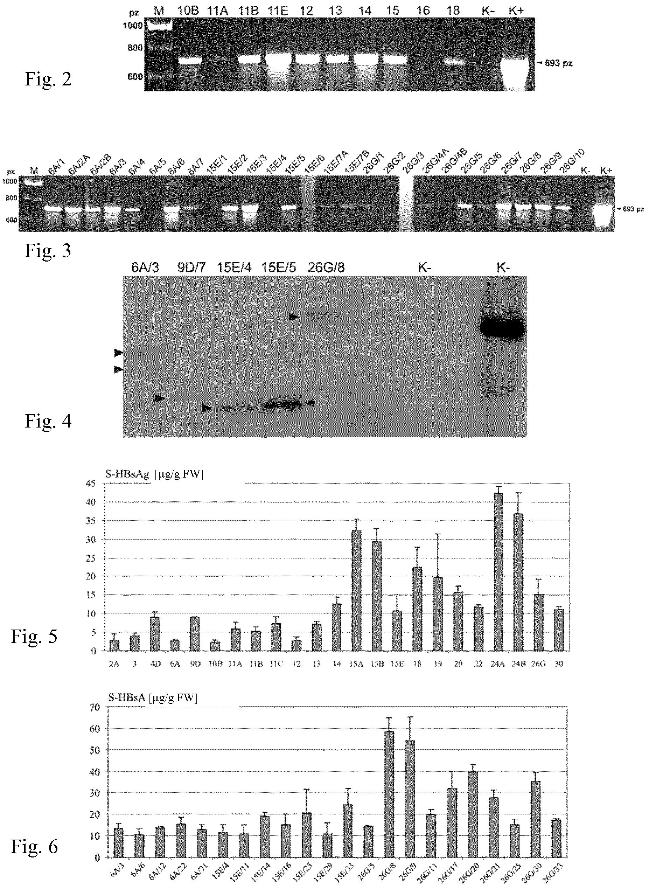

A characteristic property of the method of transforming lettuce cells and the regeneration of lettuce via organogenesis is the phosphinothricin selection system used for the first time, which significantly increases the probability of obtaining uniformly transgenic plants, i.e. not exhibiting the characteristics of chimaeras [Fig. 2]. Although a lettuce containing a herbicide resistance gene has been described in literature (Mohapatra et al. 1999), the regeneration of plants following transformation with a vector containing both the *bar* gene for phosphinothricin resistance and the *nptII* gene for kanamycin resistance was only possible following the use of antibiotic selection process. Regenerated plants according to the present invention under phosphinothricin selection conditions pass the transgene to their progeny following self-pollination according to a Mendelian ratio of 3:1, which is confirmable via PCR [Fig. 3]. In the case of weaker selective factors, mainly antibiotics such as kanamycin, there is a significant risk of regenerating plants resistant to the selection factor, but nevertheless non-transgenic (escapees) or genetically chimaeric ones. Furthermore, a selection system of transgenic lettuce based on a herbicide makes it possible to use such a product as a source material for the production of an oral vaccine in accordance with pertinent requirements and recommendations.

A significant characteristic of the transformation method used in the present invention is the ability to introduce a small number, 1 or 2 copies, of an expression cassette [Fig. 4] into the lettuce genome. A small number of copies of an expression cassette in consequence ensures the stable expression of a transgene, meaning the immunogenic S subunit protein of the HBs surface antigen in primary regenerant plants (T0 generation) and in progeny plants (T1 generation). The processes, which commonly lead to the silencing of exogenous DNA sequences introduced by way of genetic transformation have thus been significantly reduced or eliminated, despite the relative variability and rearrangements of the lettuce genome (McCabe et al. 1999). The conditions essential to obtaining a stable transformation of lettuce and the efficient expression of the transgene encompass: structural characteristics of the cassette in the binary plasmid in *Agrobacterium* as well as a method of obtaining transgenic lettuce plants. The stability of the expression level resultant from the structural characteristics of the expression cassette, as well as 1-2 copies of the cassette introduced into the plant genome should be understood as expression levels in T1 progeny similar or higher than those of those observed in the T0 generation.

The next aspect of the present invention are transformed lettuce cells and plants regenerated from them, as well as plants from subsequent generative progeny characterised by the simultaneous expression of the small surface antigen protein of HBV, S-HBsAg, and resistance to phosphinothricine herbicides.

The method of transforming and regenerating lettuce described in the present invention facilitates the production of transgenic plant lines, characterized in that they are resistant to phosphinothricin herbicides such as Basta, as well as expressing S-HBsAg at a level reaching a dozen to several dozen µg/g FW of leaves [Fig. 5]. These characteristics are maintained in the generative progeny T1 [Fig. 6] and in subsequent generative progeny. S-HBsAg produced in lettuce is characterized by its native structure and retains its ability to fold into highly immunogenic subviral or virus-like particles (VLPs), as evidenced by ELISA analyses performed using kits from the Abbot company, which themselves are based on a monoclonal antibody against epitope "a" exposed on the surface of subviral particles. Moreover, the observation of bands on western blots, which correspond to various forms of S-HBs, there were also dimers, which are the first stage in the process of VLP assembly from S-HBsAg [Fig. 7]. These particles were also observed directly in the mesophyll layer of lettuce leaves [Fig. 8] using a JEM1200EXII TEM from Jeol.

The next aspect of the present invention is the use of transgenic lettuce resistant to phosphinothricin and producing the antigenic protein S-HBs in the production of an oral vaccine against viral hepatitis type B.

Lettuce (*Lactuca sativa* L.) is a species of plant which is characterized by properties significant for the production of oral vaccines, in contrast to species used thus far. In contrast to a clear majority of agricultural plants, lettuce is amenable to direct consumption without prior processing, most importantly thermal processing. It also contains no counter-nutritional substances nor allergens, which could constitute a factor limiting its intake. A characteristic property of transgenic lettuce producing S-HBsAg and at the same time resistant to herbicides is its usefulness in and amenability to use as a raw material for the production of a vaccine against HepB to be applied orally.

The next aspect of the present invention is lyophilised plant material, its use in the production of an oral vaccine in the form of derivatives: a suspension, syrup, granulate, tablets and capsules containing the S-HBsAg vaccinating protein, and the aforementioned derivatives of the lyophilisate and a method of preparing them from plant material.

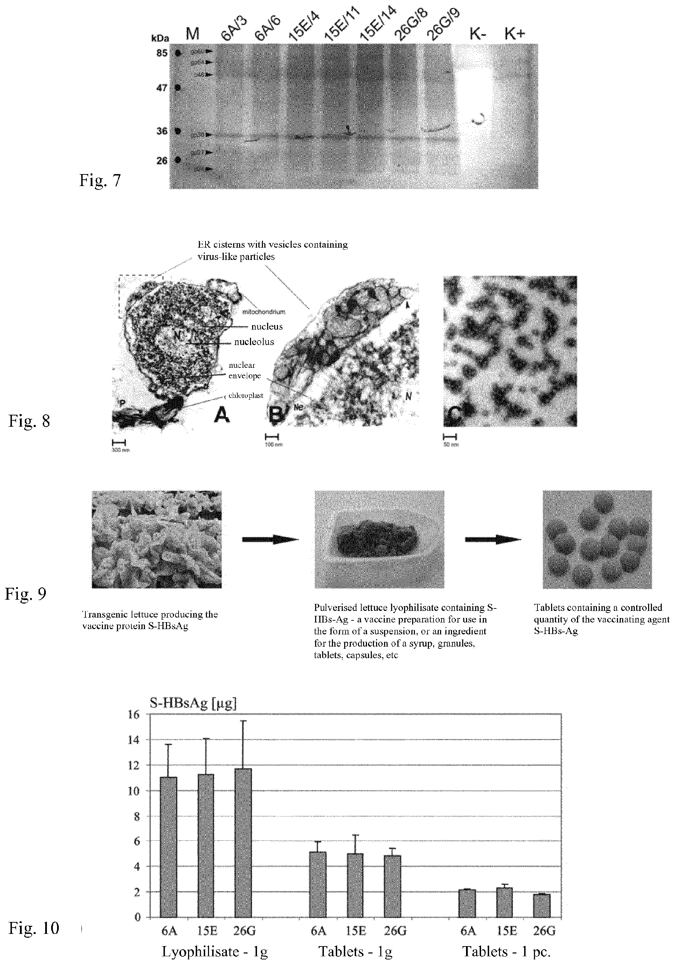

An effective oral vaccine against HepB, in contrast to solutions used to date [see literature cited] contains a condensed state of pulverised lyophilisate used in the form of a suspension, syrup as well as granulate, tablets or capsules. The raw material in the production of the condensate are lettuce leaves containing S-HBsAg, which are lyophilised. This material is pulverised, and subsequently, using physiological saline or similar buffer such as PBS, as well as the addition of ancillary substances, formed into a suspension or syrup, granulate, tablets or capsules [Figs. 9, 10]. The pulverised, lyophilised plant material as well as the suspended/syrup or granulated/tablet/capsule forms of the oral vaccine [Fig. 10], in contrast with the raw plant material are characterized by the following properties facilitating:
- preservation of plant material while maintaining a high content of the vaccinating protein, S-HBsAg
- concentration of the vaccinating substance dose, i.e the S-HBsAg protein, to a level sufficient for oral immunization
- administration of a standardized dose of S-HBsAg due to a) the control of the S-HBsAg level at various stages of preparation of the suspension or syrup as well as granulate, tablets or capsules as well as b) selection of appropriate raw material and ancillary substances
- easy storage of the pulverised lyophilisate as well as of the granulate and tablets or capsules at room temperature while maintaining the level of S-HBsAg throughout a period of at least 12 months
- a simple method of vaccinating orally through drinking or eating.

Vaccines produced according to the present invention may be used to orally vaccinate against HepB using a suspension or syrup as well as a granulate, tablets or capsules produced from a pulverised lyophilisate of lettuce containing the S-HBs antigen.

The S-HBs surface antigen of HBV is itself a strong immunogen which may, without the addition of adjuvants, elicit an immune response in a number of mammalian species, including mice, chimpanzees as well as humans. The immune response may be of the cellular type encompassing the formation of specific cytotoxic lymphocytes, or of the humoral type, with the formation of specific anti-SHBs antibodies. It is generally accepted, that in humans and chimpanzees, a humoral response at an appropriate level is generally sufficient to protect against disease upon exposure to the virus. Depending on the method of administration, the immune reaction against the S-HBs antigen encompasses the formation of IgG class antibodies (intramuscular immunization) as well as IgG and IgA with immunization via the mucous membranes of the digestive tract with oral administration, as well as through the respiratory epithelia (inhalation), intravaginally and rectally.

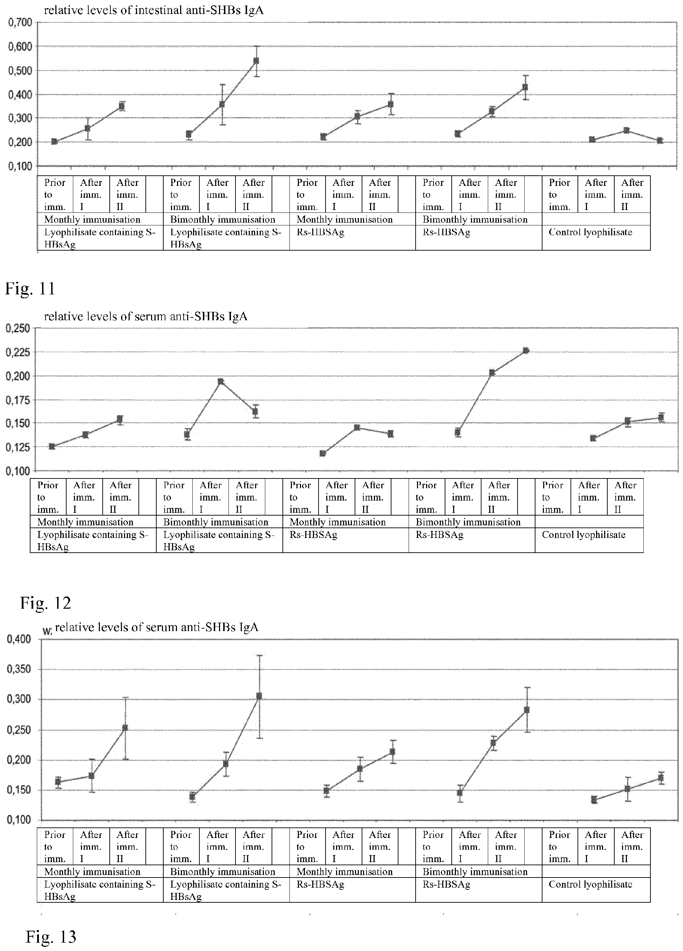

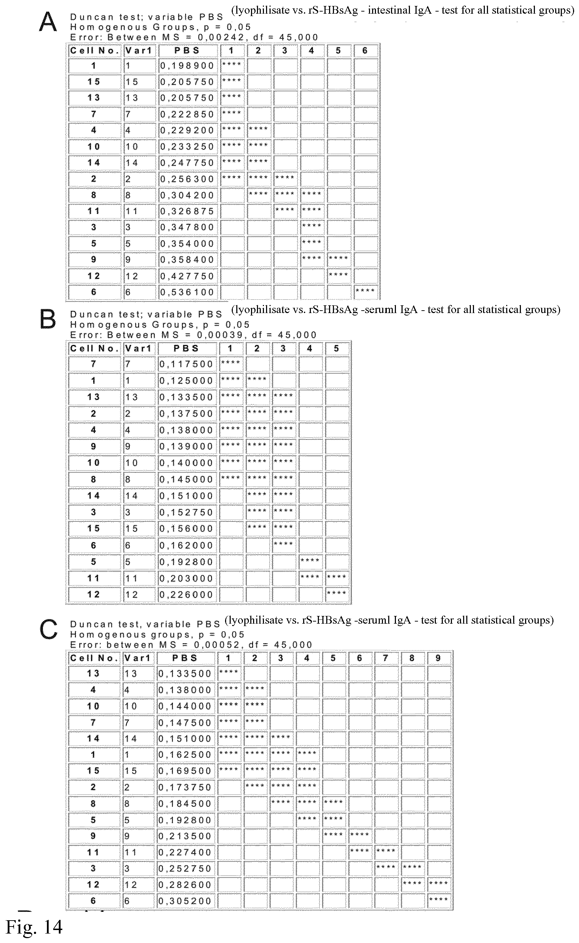

As was shown, the S-HBs antigen is immunogenic to mice following the prior preservation of plant material containing S-HBsAg via lyophilisation and resuspension immediately prior to administering (see. Example 5). The immune response against the S-HBs antigen occurs as a result of the initial oral administration of the antigen (priming), and then as a result of subsequent repeated immunizations which result in secondary responses (boosting) [Figs. 11-14]. In animals immunized orally with the S-HBs antigen present in a suspension of transgenic lettuce lyophilisate according to the present invention, we did not observe tolerance which would result in a lack of response to injected S-HBs antigen as a result of one or more prior oral immunizations. It is accepted that the oral administration of antigens induces GALT cells (GALT - gut associated lymphoid tissue), cells of the immune system associated with the digestive tract. In the GALT structure one finds Peyer's patches, which expose M cells (microfold cells) on the lumen side. M cells are characterised by the ability to effectively gather both degraded and complete proteins, viruses, bacteria or single-celled parasites. Antigens or microbiota are then transported into immune system cells, including macrophages and dendritic cells as well as T and B lymphocytes, both locally within the mucous membranes and to peripheral lymphatic organs as a result of systemic circulation. The S-HBs antigen administered orally according to the present invention may be uptaken by the M cells from the gastrointestinal tract and then, following uptake and degradation by macrophages and dendritic cells, it is presented to lymphocytes present in the gastrointestinal mucosa and elicits a local immune response in the form of the production of specific anti-SHBs antibodies of the IgA class [Figs. 11, 14A]. Within several hours, S-HBsAg can be found in the blood and thence it reaches peripheral immune organs, thereby inducing a systemic immune response with IgA and IgG antibodies [Figs. 12, 13, 14B, 14C]. An immunization method according to the present invention makes it possible to establish the size of the S-HBs antigen dose, as well as the immunization scheme, meaning the number of immunizations, as well as the period of time necessary between priming and boosting. This is possible due to the standardized preserved material, pulverised lyophilisate, available in the form of a suspension or syrup or formed into granulate/tablets/capsules. The immunization method according to the present invention encompassing a suspension/syrup and/or a granulate/tablets/capsules produced from transgenic lettuce lyophilisate facilitates the establishment of the number of antigen doses, dose size, as well as the period of time between individual immunizations depending on the age of immunized animals and humans, their overall condition and immunocompetence, sex, body mass and other parameters influencing the immune response. An immunization method according to the present invention will facilitate the induction of a humoral immune response encompassing the induction of IgG as well as IgA class antibodies, which guarantees a wider scope of immunity than the commercially available vaccine administered as an intramuscular injection. Since it is supposed that vertical transmission may encompass mucosa as a gate for infection, and the method of immunisation according to the present invention will be a potentially more stringent immune protection among the families of those infected with HBV, where otherwise vertical transmission is a real risk. The immunization method revealed will facilitate the use of the S-HBs antigen produced in transgenic lettuce as an immunomodulator of an immune response in persons being chronic HBV carriers.

A method of preparing a vaccine according to the present invention, in the form of a suspension, syrup as well as granulate, tablets or capsules, will facilitate the production of a homogenous, vaccine preparation, composed for a particular immunization method and/or the induction of an immune response, possibly with appropriate adjuvants enhancing said immune response, following antigen administration onto mucous membranes.

The revealed immunization method encompassing the administration of the vaccine preparation in the form of a suspension or syrup prepared from lyophilized material from transgenic lettuce and/or in the form of a granulate/tablets/capsules will facilitate the administration of the immunogenic S-HBs antigen in an amount of 1 or more nanograms, meaning 1-1000 ng, or several or more micrograms, i.e. 2-1000 µg or several miligrams, e.g. 2-100 mg. Following appropriate preparation, the S-HBs antigen from transgenic lettuce may be administered orally to animals or humans in a wide range of doses, as above.

To better illustrate the nature of the present invention, this description has been supplemented with a list of sequences and figures.

Sequence No. 1 (SEQ ID No. 1) represents the nucleotide sequence of the expression cassette P35S-SHBs-NOSt contained in the binary vector pKHBSBAR described in the examples and designed for the transformation of plants.

Sequence No. 2 (SEQ ID No. 2) represents the nucleotide sequence of the expression cassette PNOS-bar-g7t contained in the binary vector pKHBSBAR described in the examples and designed for the transformation of plants.

Figure 1 shows a schematic of the construction of the pKHBSBAR vector used in the transformation of lettuce, containing a sequence encoding the small surface antigen, S-HBs, of the hepatitis type B virus (HBV) under the control of the CaMV 35S RNA constitutive promoter and a nopaline synthase terminator (NOSt) as well as the sequence of the *bar* gene encoding phosphinothricin acetyltransferase under the control of the nopaline synthase promoter (PNOS) and the g7t terminator, which determines the resistance of transgenic plants to phosphinothricin herbicides.

Legend: S-HBs - the sequence encoding the small surface antigen of HBV, P35S - 35S RNA promoter of the cauliflower mosaic virus (CaMV), NOSt - nopaline synthase gene terminator, BAR - coding sequence of the *bar* gene- phosphinothricin acetyltransferase, PNOS - nopaline synthase gene promoter, g7t - g7 terminator , RB, LB - right and left flanking T-DNA sequences, NPT III - neomycin phosphotransferase gene, GUS - β-glucuronidase coding sequence, GUS-INT - β-glucuronidase coding sequence with intron.

Figure 2 shows an electrophoretic separation of the products of amplifications performed in order to analyze the presence of the S-HBs transgene in the genomic DNA of primary transformants of lettuce (T0 generation) using PCR and primers specific for the S-HBs sequence.

Electrophoretic separation lanes: M - DNA molecular mass marker (200 bp DNA Ladder, MBI Fermentas), 10B-18 - analysed plants, K- - negative control - DNA of non-transgenic plants, K+ - positive control - pKHBSBAR plasmid.

Figure 3 shows an electrophoretic separation of the products of amplifications performed in order to analyze the presence of the S-HBs transgene in the genomic DNA of progeny of lettuce transformants (T1 generation), lines 6A, 15E and 26G using PCR and primers specific for the S-HBs sequence.

Electrophoretic separation lanes: M - DNA molecular mass marker (200 bp DNA Ladder, MBI Fermentas), 6A/1-26G/10 - analysed plants, K- - negative control - DNA of non-transgenic plants, K+ - positive control - pKHBSBAR plasmid.

Figure 4 shows the result of the analysis of the number of sites of T-DNA integration in genomic DNA of lettuce transformant progeny (T1 generation) digested with the restrictase *Eco*RI and with Southern hybridisation using a probe specific for the S-HBs transgene.

Blot lanes: 6A/3-26G/8 - analysed plants, K- - negative control - DNA non-transgenic plant, K+ - positive control - pKHBSBAR plasmid digested with *Eco*R I.

Figure 5 shows a graphic analysis of S-HBsAg protein content in the leaves of primary lettuce transformants (T0 generation) using the AUSZYME® ELISA immunoenzymatic test from Abbott, specific for the S-HBs antigen. The S-HBsAg content is expressed in µg/g FW as an arithmetic mean along with the standard deviation from three trials.

Figure 6 shows a graphic analysis of S-HBsAg protein content in the leaves of progeny lettuce transformants (T1 generation), lines 6A, 15E and 26G using the AUSZYME® ELISA immunoenzymatic test from Abbott, specific for the S-HBs antigen. The S-HBsAg content is expressed in µg/g FW as an arithmetic mean along with the standard deviation from three trials.

Figure 7 shows the results of analyses of S-HBs transgene and S-HBsAg protein forms in leaves of progeny lettuce transformants (T1 generation) using Western blotting and polyclonal rabbit antibodies specific for S-HBsAg.

Blot lanes: M - molecular mass marker (MBI Fermentas), 6A/3-26G/9 - analysed plants, K- - negative control - non-transgenic plant protein extract, K+ - positive control - S-HBsAg protein from Prof. R. Schirmbeck (University of Ulm, Germany). The forms of S-HBsAg indicated are: p24 - unglycosylated 24 kDa monomer of the S-HBsAg protein, gp27 - glycosylated 27 kDa monomer of the S-HBsAg protein, gp30 - probably a glycosylated 30 kDa monomer of the S-HBsAg protein, p48 - unglycosylated 48 kDa dimer of the S-HBsAg protein, gp54 - glycosylated 54 kDa dimer of the S-HBsAg protein, gp60 - probably a glycosylated 60 kDa dimer of the S-HBsAg protein.

Figure 8 shows a micrograph of VLPs composed of the S-HBsAg protein in the cells of lettuce leaf mesophyll (panel A, B) as well as in the vaccine preparation Engerix B (SmithKline Beecham) (panel C). In the plant cells, the VLPs accumulate in somas which are then closed in the cisterns of the endoplasmic reticulum - ER. The micrographs were made using a JEM1200 EXII TEM from Jeol.

Figure 9 shows vaccines against HepB, for oral vaccination, obtained from transgenic lettuce producing the S-HBsAg protein - pulverised lyophilisate for use in the form of a suspension or syrup as well as in the form of a granulate, tablets or capsules.

Figure 10 shows a graphic analysis of S-HBsAg protein content using the ELISA kit AUSZYME® from Abbott, specific for the S-HBs antigen in: 1 g lyophilisate as well as in 1 g of tablets and 1 tablet produced from the leaves of progeny lettuce transformants (T1 generation) from the lines 6A, 15E and 26G. S-HBsAg content is expressed in *µ*g as an arithmetic mean with standard deviation from 3 determinations.

Figure 11 shows a graph comparing the immune response of mucous membranes of the gastrointestinal tract in terms of IgA production, induced by the S-HBsAg antigen contained in a suspension of transgenic lettuce lyophilisate as well as by the Engerix B vaccine (SmithKline Beecham) (rS-HBsAg). The antigen was administered *per os* to BALB/c mice at 100 ng/100*µ*l/animal at 1 or 2 month intervals. Control mice received a suspension of non-transgenic lettuce lyophilisate. The graph is a plot of averages with standard deviations of titres expressed in mIU/ml of anti-SHBs IgA prior to immunization, 10 days after and 10 days after the second immunization for five mice in each experimental and control group.

Figure 12 shows a graph comparing systemic immune responses in terms of serum IgA levels, induced using S-HBsAg contained in a suspension of transgenic lettuce lyophilisate as well as the Engerix B vaccine preparation (SmithKline Beecham) (rS-HBsAg). The antigen was administered *per os* to BALB/c mice at 100 ng/100*µ*l/animal at 1 or 2 month intervals. Control mice received a suspension of non-transgenic lettuce lyophilisate. The graph is a plot of averages with standard deviations of titres expressed in mIU/ml of anti-SHBs IgA prior to immunization, 10 days after and 10 days after the second immunization for five mice in each experimental and control group.

Figure 13 shows a graph comparing systemic immune responses in terms of serum IgG levels, induced using S-HBsAg contained in a suspension of transgenic lettuce lyophilisate as well as the Engerix B vaccine preparation (SmithKline Beecham) (rS-HBsAg). The antigen was administered *per os* to BALB/c mice at 100 ng/100*µ*l/animal at 1 or 2 month intervals. Control mice received a suspension of non-transgenic lettuce lyophilisate. The graph is a plot of averages with standard deviations of titres expressed in mIU/ml of anti-SHBs IgG prior to immunization, 10 days after and 10 days after the second immunization for five mice in each experimental and control group.

Figure 14 shows a statistical analysis (Duncan test) of the significance of changes in anti-SHBs antibody levels in terms of mucous membrane IgA (panel A), as well as serum IgA (panel B) and IgG (panel C) as a result of the immune response in mice following the oral administration of S-HBs antigen in a suspension of transgenic lettuce lyophilisate as well as the rS-HBsAg antigen from the Engerix B vaccine (SmithKline Beecham). Each statistical group was given a sequential number, and groups not showing statistically significant differences were marked with an asterisk.

Group markings: 1 - mice immunized with S-HBsAg (lyophilisate) at monthly intervals, anti-SHBs antibody levels prior to immunisation, 2 - mice immunized with S-HBsAg (lyophilisate) at monthly intervals, anti-SHBs antibody levels following the first immunization, 3 - mice immunized with S-HBsAg (lyophilisate) at monthly intervals, anti-SHBs antibody levels following the second immunization, 4 - mice immunized with S-HBsAg (lyophilisate) at bimonthly intervals, anti-SHBs antibody levels prior to immunisation, 5 - mice immunized with S-HBsAg (lyophilisate) at bimonthly intervals, anti-SHBs antibody levels following the first immunization, 6 - mice immunized with S-HBsAg (lyophilisate) at bimonthly intervals, anti-SHBs antibody levels following the second immunization, 7 - mice immunized with rS-HBsAg (Engerix B) at monthly intervals, anti-SHBs antibody levels prior to immunisation, 8 - mice immunized with rS-HBsAg (Engerix B) at monthly intervals, anti-SHBs antibody levels following the first immunization, 9 - mice immunized with rS-HBsAg (Engerix B) at monthly intervals, anti-SHBs antibody levels following the second immunization, 10 - mice immunized with rS-HBsAg (Engerix B) at bimonthly intervals, anti-SHBs antibody levels prior to immunisation, 11 - mice immunized with rS-HBsAg (Engerix B) at bimonthly intervals, anti-SHBs antibody levels following the first immunization, 12 - mice immunized with rS-HBsAg (Engerix B) at bimonthly intervals, anti-SHBs antibody levels following the second immunization, 13 - mice given control lyophilisate, anti-SHBs antibody levels prior to lyophilisate administration, 14 - mice given control lyophilisate, anti-SHBs antibody levels following the first lyophilisate administration, 15 - mice given control lyophilisate, anti-SHBs antibody levels following the second lyophilisate administration.

The following examples are given solely to better illustrate individual aspects of the present invention and should not be seen as its entire scope, as defined in the Claims. Example 1. Construction of the vector pKHBSBAR for transforming lettuce.

The preparation of the vector containing the sequence encoding the antigen protein S-HBs under the control of the 35S promoter encompassed the following stages:
Using PCR on a template of whole genomic DNA of *Agrobacterium tumefaciens* of the nopaline strain C58, we amplified the nopaline synthase terminator (NOSt) (Croy 1993). At the same time, we introduced the following restriction sites into the NOSt: *Pst* I, *Xho*I at the 5' end as well as *Hin*dIII at the 3' end. The terminator was cloned into the plasmid pUC18 (MBI Fermentas, Yanisch-Perron et al. 1985, Genebank L09136) yielding p18PNOSt, which was then sequenced.

The previously cloned 35S promoter of CaMV (P35S) from the p35SGUS-INT vector (Vannaceyt et al. 1990) was cloned into the pBluescript KS vector (Stratagene, Alting-Mees and Short 1989, Genebank X52327) removing the *Pst*I restriction site at the 5' end of the promoter by *Pst*I digestion and 3' sticky end degradation using T4 DNA polymerase, and thence by re-ligation finally yielding the plasmid pKSP35SGI.

The 35S promotor from pKSP35SGI was cloned into p18PNOSt which yielded the pMG2A vector.

Using PCR, we amplified the coding sequence of S-HBs (bases 157 - 837) using the previously obtained plasmid, pHBV312, as a template which contains the complete genome of the Polish HBV viral isolate (Plucienniczak 1994). At the same time, the S-HBs sequence was supplemented by the following restriction sites *Bam*HI at the 5' end and *Pst*I at the 3' end. The modified S-HBs sequence was cloned into pGEM-T (Promega, Marcus et al. 1996) yielding pGTHBS, which was then sequenced.

The S-HBs sequence from pGTHBS was then cloned into the pMG2A vector, yielding pMG2AHBS.

The complete expression cassette, P35S-S-HBs-NOSt, was then transferred into the vector pGPTV-BAR (Becker et al. 1992) simultaneously removing the fragment GUS-NOSt, yielding the pKHBSBAR vector.

The vector pKHBSBAR was prepared using restrictases, Taq DNA polymerase and other reagents from MBI Fermentas. A schematic of the construction of the binary vector pKHBSBAR is given in detail [Fig. 1].

The completed binary vector was introduced into *Agrobacterium tumefaciens* cells of the strain EHA105. The presence of the plasmid in *Agrobacterium* clones was verified using PCR using primers specific for the S-HBs antigenic protein coding sequence. Example 2. Transformation of lettuce using *Agrobacterium tumefaciens.*

In order to carry out the transformation procedure we prepared appropriate explants of lettuce (Polish variety Syrena) as well as the *Agrobacterium tumefaciens* strain EHA105 containing the plasmid pKHBSBAR.

To germinate the lettuce, seeds of leafy lettuce (var. Syrena) were sterilized for 12 minutes in 20% Clorox® bleach containing 0.01% Tween® 20, and then rinsing 5-6 times in sterile deionised water in order to remove sterilising solution residues. The seeds were allowed to germinate in a 16h light/8h dark photoperiod. The source material for transformation were 2-3 mm cotyledons isolated from 2 - 3 day-old sprouts. For use in the transformation, *Agrobacterium tumefaciens* strain EHA105 containing the plasmid pKHBSBAR was sown onto the YEB agar medium (Vervliet et al. 1975) with 50 mgl⁻¹ kanamycin and rifampicin at 100 mgl⁻¹, and then re-inoculated onto AB minimum medium (Chilton et al. 1974) with antibiotics as above. The bacterial culture was maintained in darkness at 28°C. From the minimum medium the bacteria were inoculated onto MG/L liquid medium (Garfinkel and Nester 1980). The liquid cultures were shaken at around 250 RPM at 28°C. When the bacterial culture reached the logarithmic growth phase (OD₅₅₀= 0.4-0.8), 1 ml of bacterial suspension was extracted from the culture and used to inoculate 100 ml of fresh MG/L medium. The *Agrobacterium* was cultured again until reaching OD₅₅₀ = 0.4-0.8. Next, using standard microbiological procedures, the *Agrobacterium* was diluted to a density of 10⁸ cells/ml. To do this, the cell suspension was centrifuged for 10 min at 10 KRPM and 4°C. The bacteria were then suspended in MSGA, containing macro-and microelements of MS medium (Murashige and Skoog 1962), 5% glucose and 100 nM acetosyringone in a volume sufficient to obtain 10⁸ cells/ml. The isolated lettuce cotyledon explants were incubated directly in the *Agrobacterium* cell suspension. The explants were inoculated in the bacterial suspension in a dish for about 10 - 15 min., and then to co-culture them, the *Agrobacterium* was roughly removed from the explants which were then placed onto regenerative LR1 medium containing: macro- and microelements and vitamins according to the MS medium, saccharose 3%, agar 0.8%, 6-benzylaminepurine 0.2 mgl⁻¹, α-naphthylacetic acid 0.05 mgl⁻¹, pH 5.75. The *Agrobacterium* and lettuce cotyledon co-culture was maintained for 4 days in darkness at about 24°C. The explants were then rinsed in sterile deionised water and transferred onto fresh LR1 selection medium containing the antibiotic timentin (Smith Kline Beecham) at 300 mgl⁻¹ and phosphinothricin (Riedel de Haën), the active substance in the herbicide Basta at a concentration of 2.5 mgl⁻¹. The plant explants were maintained at a temperature of about 24°C during the incubation in a day/night 16/8h photoperiod and a light intensity of 3000 - 4000 lx. The explants growing on LR1 medium were re-inoculated onto fresh medium initially every 5 days and after a month of culturing every 2 weeks. During the 6-8 week culture period on LR1 selective medium, we observed the formation of a morphogenic callus with meristematic centers as well as initial shoot regeneration in the form of rosettes and leaf buds. Regenerating transgenic tissue was then transferred onto LR2 selective medium containing: macro- and microelements according to the SH medium (Schenk and Hildebrandt 1972), vitamins B5 (Gamborg et al. 1968), saccharose - 3%, agar - 0.7%, kinetin - 0.5 mgl⁻¹, zeatin - 0.5 mgl⁻¹, pH 5.75, timentin - 150 mgl⁻¹ and phosphinothricin - 2.5 mgl⁻¹. Transgenic plants developing on LR2 selective medium after about 8 - 10 weeks were cut and transplanted onto 1/2SH selective medium containing half the macroelements and a full dose of the microelements of SH medium, vitamins B5, saccharose - 3%, agar - 0.8%, pH 5.75, as well as timentin and phosphinothricin as above. Transgenic plants which took root on 1/2SH selective medium were transferred into *ex vitro* conditions and adapted to soil conditions, meaning gardening soil in a phytotron (16/8h day/night photoperiod, 22°C, lighting at plant level about 15 - 20 klx). The transformation procedure, being the subject of the present invention, facilitates production of transgenic lettuce resistant to phosphinothricin with about 20% efficiency. The salts, growth and development regulators as well as other reagents for plant and bacterial media were purchased from POCh, Sigma as well as Difco.

Lines of potentially transgenic lettuce plants were analysed using PCR in order to initially select the presence of the transgene sequence in the genomic DNA [Fig. 2]. Progeny plants, T1 generation, were also analysed via PCR in order to determine/confirm the mendelian 3:1 inheritance mechanism of the transgene by transgenic plants [Fig. 3]. Next, the genomic DNA of progeny was hybridized with a probe complementary to the sequence of the S-HBs transgene using the Southern blot protocol [Fig. 4] in order to determine the number of integration sites of the T-DNA containing transgene. Regenerated plants, T0, as well as progeny of the T1 generation were also examined to test for the expression of the HBs surface antigen using Western blotting [Fig. 7] as well as the qualitative and quantitative sandwich ELISA test using the AUSZYME® kit from Abbott [Figs. 5, 6]. The presence of the S-HBs antigen formed into VLPs in mesophyll cells was visualised using a TEM (JEM 1200EXII from Jeol) [Fig. 8]. In order to visualise the VLPs we used standard serial section and contrasting techniques with minor modifications (Kocjan et al. 1996).

### Example 3. Confirmation of S-HBs antigen in transgenic lettuce using Western blotting.

The S-HBs antigen was confirmed in transgenic lettuce using Western blotting and the monoclonal antibody (Mab) C86132M (Biodesign) as well as polyclonal antibodies from rabbit serum. The anti-SHBs rabbit serum was obtained from Prof. B. Szewczyk (University of Gdańsk) following a triple immunisation of a New Zealand large rabbit with S-HBs from the Engerix B vaccine (Smith Kline Beecham).

In order to perform the Western blots we prepared plant extracts by grinding lettuce leaf samples in five volumes (1 mg = 5 *µ*l) of PBS with 0.5% Tween® 20. The ground samples were mixed with denaturing sample buffer (Laemmli 1970) with 50 mM DTT and incubated for 15 min. at 65°C. The extract samples along with a protein marker for S-HBs (from Prof. R. Schirmbeck, University of Ulm, Germany) as well as a protein molecular mass marker (MBI Fermentas) was loaded onto a 12.5% denaturing PAGE gel and electrophoresis was performed in the Laemmli buffer system. The proteins were transferred onto a nitrocellulose membrane using the "wet" electrotransfer method in a Bio-Rad cell. Following a triple wash in TBST (TBS with 0.05% Tween® 20), the membrane with proteins was blocked in 3% BSA in TBS. After washing in TBST, the membrane was incubated in the following mixture in TBS: 1 *µ*g/ml Mab C86132M (Biodesign) or 2000× dilution of rabbit anti-serum. Following a triple rinse in TBST, membrane was incubated in a secondary antibody solution tagged with horseradish peroxidase in TBS: 10000× dilution of goat anti-mouse polyclonal "whole molecule" antibodies (Sigma), or 10000× dilution of goat anti-rabbit polyclonal "whole molecule" antibodies (Sigma). After a quintuple rinse in TBST, the membrane was incubated with diaminobenzidine (DAB), a substrate for HRP (Sigma). All incubations were performed with agitation at about 100 RPM.

The Western bands observed correspond to different forms of S-HBs, meaning glycosylated and unmodified monomers and dimers, which are the initial stages in the production of VLPs from S-HBsAg [Fig. 7].

Buffer salts, BSA etc. were purchased from POCh as well as Sigma.

### Example 4. Determination of S-HBs antigen content in transgenic lettuce using ELISA.

The content of S-HBs antigen in leaves was determined via ELISA using the commercial kit Auszyme® Monoclonal Diagnostic Kit from Abbott. Detection using this kit is based on indication, using a monoclonal antibody, of epitope "a" of the S-HBs antigen, which is exposed on the surface of the S-HBs protein when folded into VLPs. The Auszyme® Monoclonal Diagnostic Kit from Abbott thus makes it possible to indicate the level of S-HBsAg formed into immunogenic VLPs in examined samples.

In order to determine the level of S-HBsAg in transgenic lettuce, we prepared plant extracts by grinding leaf samples in gradually added buffer, to a volume equal to a 50-fold mass of the sample. i.e. 1 mg of leaves were ground in 50 *µ*l of buffer. The following extraction buffer was used: 137 mM NaCl, 2.7 mM KCl, 8 mM Na₂HPO₄, 1.5 mM KH₂PO₄, 10.3 mM Na₂SO₃, 2% PVP40000, 0.2% BSA, 1% Tween® 20, pH = 7.4. The homogenized samples were centrifuged for 5 min. at 10000 RPM and RT. We sampled 5 - 10 *µ*l of extract from the supernatant, added 40 - 100 volumes of PBS, and then 200 *µ*l of the diluted extract was transferred into a reaction tube from Abbott. In the extract tube we placed a polystyrene sphere from the kit which was coated with a monoclonal anti-SHBs antibody against epitope "a", whereafter the mixture was supplemented with 50 *µ*l of the above antibody conjugated with peroxidase. The mixture was incubated at 28°C for 16h. The immunological reaction was halted by rinsing six times in distilled water, and 300 *µ*l o-phenyldiamine hydrochloride (OPD) solution in the kit's reaction buffer was added. After a 30 min. incubation, the peroxidase reaction was stopped using 300 *µ*l 1 N H₂SO₄. The absorbance of the coloured product was measured using a spectrophotometer at X = 492 nm. The values obtained were used to calculate the level of S-HBsAg in *µ*g/g FW of leaves according to the formula: S-HBs = [(A492 - a)/b] × dilution, where a,b are directional coefficients of the calibration curve and dilution is 2000 - 5000 × [Figs. 6, 7].

The reagents for the analyses were purchased from POCh and Sigma, with the exception of the kit from Abbot.

### Example 5. Preparation of the lyophilisate and tablets containing the vaccinating antigen S-HBs from transgenic lettuce leaves.

Plants from selected transgenic lettuce lines, characterised by relatively high S-HBsAg content, i.e. above 15 *µ*g/g FW were reared in greenhouses under natural photoperiod conditions at 20 - 22°C during the day and 14 - 16°C at night. Leaves from well-developed plants were collected, frozen in liquid nitrogen with partial homogenization, and then maintained at -80°C. The frozen material was placed in a BETA 1-16 from CHRIST® and lyophilised for 24-36 h in 0.2 mbar of vacuum at -55°C and shelves at the same temperature, on which the material was stacked in plastic trays. Lyophilised material was pulverised and stored in tightly sealed containers in the presence of silica gel as a desiccator until tablet manufacture [Fig. 9].

The powdered product was supplemented with a filler, lactose (Meggle), in a 1:1 ratio and a binder, 10% polyvinylpyrrolidone (PVP) (BASF) in CH₂Cl₂ (Merck). The ingredients were mixed in a mixer/crusher (ERWEKA) until homogeneity, from which a granulate was prepared in an oscillatory granulator (ERWEKA) through a φ 1.6 mm grid. The granulate was dried at room temperature and ground through a φ 1 mm grid. The prepared granulate was mixed with a lubricating substance, 2% magnesium stearate (Mosselman), in a rhomboidal tumble mixer (ERWEKA). Tablet cores were then minted on a tablet press (KORSCH) with φ 12 mm concave forms. The cores produced were spray-coated in a pelleting drum (ERWEKA) with individual coats of the coating solution containing: 10% cellulose acetophthalate (Colorcon), 0.5% castor oil, 89.5% acetone (POCh), with each coat being dried with a stream of air. The tablets were then coated with 20% PEG6000 (Merck) in acetone. Average tablet mass was 510 mg ± 2%.

The conversion of plant material into tablets was controlled by determining S-HBs antigen content in lyophilisate as well as in tablets [Fig. 10] according to Example 4. Tablets prepared according to the present invention, contained around 2 *µ*g S-HBsAg ea. The S-HBs antigen level in the tablets remained constant for at least 12 months.

### Example 6. Induction if a mucosal immune response in mice following the gastric administration of lettuce lyophilisate containing the S-HBs antigen.

The ability of the S-HBs antigen produced in lettuce to immunise trans-mucosally and to induce both a local immune response in the mucosa of the gastrointestinal tract as well as a systemic response was confirmed experimentally on animals.

Mice were immunised through mucosa using the lyophilised vaccinating plant material containing the S-HBs antigen. As a positive control, we used the Engerix B vaccine (SmithKline Beecham), a standard recombinant vaccine against HepB produced in yeast. Whereas the negative control was lyophilised plant material from non-transgenic lettuce var. Syrena.

The research was performed on 6 - 8 week-old inbred BALB/c mice. For the mucosal immunisation we used a dose of 100 ng S-HBs antigen/animal. For the plant material, we suspended in 100 *µ*l of PBS an appropriate amount of pulverised lyophilisate, usu. 9 - 10 mg, containing about 100 ng S-HBsAg. Control mice received 10 mg of non-transgenic lettuce suspended in 100 *µ*l PBS. In the case of the commercial vaccine, Engerix B (SmithKline Beecham), we diluted an appropriate amount of the preparation (0.1 *µ*l) in 100 *µ*l of PBS. The lyophilisate suspension or diluted Engerix B was administered gastrically via a gastric tube.

A double immunisation scheme was used: 1/ Immunisation using vaccinating lyophilisate at 1 month intervals, 2/ Immunisation using vaccinating lyophilisate at 2 month intervals, 3/ Immunisation using Engerix B (rS-HBsAg) at 1 month intervals, 4/ Immunisation using Engerix B (rS-HBsAg) at 2 month intervals. Each control or experimental group consisted of 5 animals. After 10 days following the immunization, blood and faeces were collected from the animals. IgA-class antibodies were extracted from the faeces. The faecal sample was suspended in five volumes of PBS, incubated for 15 min., and then thoroughly ground. After 10 minutes of incubation, the suspension was shaken for a further 15 min. and centrifuged for 10 minutes at 14000 RPM and 4°C. All stages of the extraction from mouse faeces were performed on ice. The supernatant containing IgA was stored at -20°C. The faeces extracts were used to detect specific anti-SHBs antibodies [Figs. 11, 14A]. Blood was syringed from the caudal artery of anaesthetised mice. The blood was centrifuged for 10 min. at 8000 RPM and 4°C. The obtained sera were stored at -20°C. The titre of specific anti-SHBs antibodies, both IgA [Figs. 12, 14B] as well as IgG [Figs. 13, 14C] were determined. IgA and IgG anti-SHBs antibodies were determined immunoenzymatically using established protocols. ELISA tests were performed in Nunc-Immuno Plate F96 Polysorp plates (NUNC™) using a harvester and reader from Bio-Rad. The plate was coated overnight at 4°C with the S-HBs antigen R86872 recombined in yeast (Biodesign) in PBS pH 7.4 at 1 µg/ml. The plate was then washed 3 times in PBST pH 7.4 (PBS with 0.05% Tween® 20), and then blocked for 90 minutes at 25°C using 5% skim milk in PBS pH 7.4. The plate was rinsed as above and the mouse sera were added which were then diluted 1/1 with PBS in order to receive 20×, 40×, 80× and 160× dilutions respectively. The plate was incubated for 45 min. at RT on a shaker set at 400 RPM. The reagents were pipetted off, the plate was washed as above and then goat "whole molecule" anti-mouse IgG conjugated with alkaline phosphatase (Sigma), or anti-mouse IgA conjugated with alkaline phosphatase (Sigma) were added at a 3000× dilution in PBS. The plate was incubated for 45 min at room temperature on a shaker as above, and then, following removal of the reagents and three washes, p-nitrophenyl phosphate (Sigma), a substrate for AP was added. A majority of the reagents were added at 100 µl/well, with the exception of the blocker and rinses, which were added at 400 µl. After one hour and stopping the reaction, absorbance was read at 405 nm using a Model 680 microplate reader from Bio-Rad. Anti-SHBs antibody titres were calculated using the reader's own software standardised against a calibration curve. The analyses were performed twice. The antibody titre, expressed in mIU/ml against a standard serum was calculated as an arithmetic mean with standard deviation for five mice. The statistical analysis of anti-SHBs antibody levels, performed using Statistica 6® software, indicates a comparable or higher immunogenicity of the S-HBs antigen from plant material according to the present invention in comparison to the commercial Engerix B vaccine using oral immunization [Fig. 14]. Significant increases in anti-SHBs antibody levels in mice occurred both in relation to the state prior to immunisation, and in relation to the controls. The greater immunogenicity of the S-HBs antigen from lyophilisate than that of rS-HBsAg from the Engerix B vaccine was observed both in terms of IgA in the intestine using 2 month intervals between immunizations [Fig. 14A], and for serum IgG with 1 month immunization intervals [Fig. 14C]. In the remaining cases we observed a comparable level of immune response to S-HBsAg from plant material and to rS-HBsAg from the Engerix B vaccine.

### Literature

1. Alting-Mees MA, Short JM (1989) pBluescript II: gene mapping vectors. Nucleic Acids Res 17: 9494.
2. Becker D, Kemper E, Schell J, Masterson R (1992) New plant binary vectors with selectable markers located proximal to the left T-DNA border. Plant Mol Biol 20: 1195 - 1197.
3. Cooreman MP, Leroux-Roels G, Paulij WP (2001) Vaccine- and hepatitis B immune globulin-induced escape mutations of hepatitis B virus surface antigen. J Biomed Sci 8: 237-247.
4. Chilton M-D, Currier TC, Farrand SK, Bendich AJ, Gordon MP, Nester EW (1974) Agrobacterium tumefaciens DNA and PS8 Bacteriophage DNA Not Detected in Crown Gall Tumors. Proc Natl Acad Sci USA 71: 3672 - 3676.
5. Croy RRD (1993) Plant selectable genes, reporter genes and promoters. W: Plant Molecular Biology Labfax. Wyd. Hames BD, Rockwood D, Croy RRD. Academic Press, Oxford. str. 175 - 178.
6. Ehsani P, Khabiri A, Domansky NN (1997) Polypeptides of hepatitis B surface antigen produced in transgenic potato. Gene 190: 107 - 111.
7. Gamborg OL, Miller RA, Ojima K (1968) Nutrient requirements for suspension cultures of soybean root cells. Exp Cell Res 50: 151-158.
8. Garfinkel DJ, Nester EW (1980) Agrobacterium tumefaciens Mutants Affected in Crown Gall Tumorigenesis and Octopine Catabolism. Journal of Bacteriology 144: 732 -743.
9. Genebank, pUC18c cloning vector (beta-galactosidase mRNA on complementary strand), Accession No. L09136.
10. Genebank, pBluescript II KS (+) vector DNA, phagemid excised from lambda ZAPII, Accession No. X52327.
11. Hollinger BF (1996) Hepatitis B Virus. W: Fields Virology, 3rd Edition. Wyd. Fields BN, Knipe DM, Howley PM et al. Lippincott - Raven Publishers. str. 2739 - 2807.
12. Huang X, Lu D, Ji G, Sun Y, Ma L, Chen Z, Zhang L, Huang J, Yu L (2004) Hepatitis B virus (HBV) vaccine-induced escape mutants of HBV S gene among children from Qidong area, China. Virus Res 99: 63 -68.
13. Huang Z, Elkin G, Maloney BJ, Buehner N, Arntzen CJ, Thanavala Y, Mason HS (2005) Virus-like particles expression and assembly in plants: hepatitis B and Norwalk viruses. Vaccine 23: 1851 - 1858.
14. Imani J, Berting A, Nitsche S, Schaefer S, Gerlich WH, Neumann K-H (2002) The integration of a major hepatitis B virus gene into cell-cycle synchronized carrot cell suspension cultures and its expression in regenerated carrot plants. Plant Cell Tiss Org 71: 157 - 164.
15. Joung YH, Youm JW, Jeon JH, Lee BC, Ryu CJ, Hong HJ, Kim HC, Joung H, Kim HS (2004) Expression of the hepatitis B surface S and preS2 antigens in tubers of Solanum tuberosum. Plant Cell Rep 22: 925 - 930.
16. Kapusta J, Modelska A, Figlerowicz M, Pniewski T, Letellier M, Lisowa O, Yusibov V, Koprowski H, Plucienniczak A, Legocki AB (1999) A plant-derived edible vaccine against hepatitis B virus. FASEB J 13: 1796 - 1799.
17. Kapusta J, Modelska A, Figlerowicz M, Pniewski T, Lisowa O, Koprowski H, Plucienniczak A, Legocki AB (2000) Plant-based edible vaccine against HBV. Immunology Letters 73: 269.
18. Kapusta J, Modelska A, Pniewski T, Figlerowicz M, Jankowski K, Lisowa O, Plucienniczak A, Koprowski H, Legocki AB (2001) Oral immunization of human with transgenic lettuce expressing hepatitis B surface antigen. Adv Exp Med Biol 495: 299 - 303.
19. Kocjan G, Samardakiewicz S, Woźny A (1996) Regions of lead uptake in Lemna minor plants and localization of this metal within selected parts of the root. Biologia Plantarum 38: 107 - 117.
20. Kong Q, Richter L, Yang YF, Arntzen CJ, Mason HS, Thanavala Y (2001) Oral immunization with hepatitis B surface antigen expressed in transgenic plants. Proc Natl Acad Sci USA 98: 11539 - 11544.
21. Laemmli UK (1970) Cleavage of structural proteins during the assembly of the head of bacteriophage T4. Nature 227: 680 - 685.
22. Lam DM, Arntzen CJ (1996) Vaccines produced and administered through edible plants. US Patent 5 484 719.
23. Marcus L, Hartnett J, Storts DR (1996) The pGEM®-T and pGEM®-T Easy Vector Systems. Promega Notes Magazine 58: 36.
24. Mason HS, Lam DM-K, Arntzen CJ (1992) Expression of hepatitis B surface antigen in transgenic plants. Proc Natl Acad Sci USA 89: 11745-11749.
25. Mason HS, Thanavala Y, Arntzen CJ, Richter E (2003) Expression of immunogenic hepatitis B surface antigen in transgenic plants. US patent 6 551 820 B1
26. McCabe MS, Mohapatra UB, Debnath SC, Power JB, Davey MR (1999) Integration, expression and inheritance of two linked T-DNA marker genes in transgenic lettuce. Molecular Breeding 5: 329 - 344.
27. Mohapatra U, McCabe MS, Power JB, Schepers F, Van der Arend A, Davey MR (1999) Expression of the bar gene confers herbicide resistance in transgenic lettuce. Transgenic Res 8: 33 - 44.
28. Murashige T, Skoog F (1962) A revised medium for rapid growth and bioassays with tobacco tissue cultures. Physiol Plantarum 15: 473 - 479.
29. Plucienniczak A (1994) Molecular cloning and sequencing of two complete genomes of Polish isolates of Human Hepatitis B Virus. Genebank, Accesion No. Z35716, Z35717.
30. Pniewski T, Kapusta J, Plucienniczak A (2006) Agrobacterium-mediated transformation of yellow lupin to generate callus tissue producing HBV surface antigen in a long-term culture. J Appl Genet 47: 309 - 318.
31. Richter LJ, Thanavala Y, Arntzen CJ, Mason HS (2000) Production of hepatitis B surface antigen in transgenic plants for oral immunization. Nature Biotechnology 18: 1167-1171.
32. Rukavtsova EB, Zolova OE, Buryanova NYa, Borisova VN, Bykov VA, Buryanov YaI (2003) Analysis of Transgenic Tobacco Plants Carrying the Gene for the Surface Antigen of the Hepatitis B Virus. Russian Journal of Genetics 39: 41-45.
33. Schenk RU, Hildebrandt AC (1972) Medium and techniques for induction and growth of monocotyledonous and dicotyledonous plant cell cultures. Can Journal of Botany 50: 199 - 204.
34. Shulga NYa, Rukavtsova EB, Krymsky MA, Borisova VN, Melnikov VA, Bykov VA, Buryanov YaI (2004) Expression and Characterization of Hepatitis B Surface Antigen in Transgenic Potato Plants. Biochemistry (Moscow) 69: 1158 - 1164.
35. Smith ML, Mason HS, Shuler ML (2002) Hepatitis B Surface Antigen (HBsAg) Expression in Plant Cell Culture: Kinetics of Antigen Accumulation in Batch Culture and Its Intracellular Form. Biotechnology and Bioengineering 80: 812 - 822.
36. Sojikul P, Buehner N, Mason HS (2003) A plant signal peptide-hepatitis B surface antigen fusion protein with enhanced stability and immunogenicity expressed in plant cells. Proc Natl Acad Sci USA 100: 2209 - 2214.
37. Sunil Kumar GB, Ganapathi TR, Revathi CJ, Prasad KSN, Bapat VA (2003a) Expression of hepatitis B surface antigen in transgenic banana plants and NT-1 cell line of tobacco. BARC News Lett 237: 85 - 96.
38. Sunil Kumar GB, Ganapathi TR, Revathi CJ, Prasad KSN, Bapat VA (2003b) Expression of hepatitis B surface antigen in tobacco cell suspension cultures. Prot Exp Purif 32: 10 - 17.
39. Sunil Kumar GB, Ganapathi TR, Revathi CJ, Srinivas L, Bapat VA (2005) Expression of hepatitis B surface antigen in transgenic banana plants. Planta 222: 484 - 493
40. Sunil Kumar GB, , Srinivas L, Ganapathi TR, Bapat VA (2006) Hepatitis B surface expression in transgenic tobacco (Nicotiana tabacum) plants using four different expression cassettes. Plant Cell Tiss Org 84: 315 - 323.
41. Sunil Kumar GB, Ganapathi TR, Srinivas L, Revathi CJ, Bapat VA (2006) Expression of hepatitis B surface antigen in potato hairy roots. Plant Sci 170: 918 - 925.
42. Thanavala Y, Yang Y-F, Lyons P, Mason HS, Arntzen CJ (1995) Immunogenicity of transgenic plant-derived hepatitis B surface antigen. Proc Natl Acad Sci USA 92: 3358 -3361.
43. Vannaceyt G, Schmidt R, O'Connor-Sanchez A, Willmitzer L, Rocha-Sosa M (1990) Construction of an intron-containing marker gene: Splicing of the intron in transgenic plants and its use in monitoring early events in Agrobacterium - mediated plant transformation. Mol Gen Genet 220: 245 - 250.
44. Vervliet G, Holsters M, Teuchy H, van Montagu M, Schell J (1975) Characterisation of different plaque - forming and defective temperate phages in Agrobacterium strains. J Gen Virol 26: 33 - 48.
45. Walewska-Zielecka B, Swiderska H, Plucienniczak G, Jończyk M, Nitkiewicz J, Plucienniczak A, Nowoslawski A (1996) Etiology of chronic hepatitis as evaluated by liver biopsy and serum samples submitted to the Department of Immunopathology of the National Institute of Hygiene in 1993-1995. Przegl 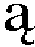 d Epidemiologiczny 50: 353 - 363.
46. Yanisch-Perron C, Vieira J, Messing J (1985) Improved M13 phage cloning vectors and host strains: nucleotide sequences of the M13mp18 and pUC19 vectors. Gene 33: 103-119.
47. Young MD, Rosenthal MH, Dickson B, Du W, Maddrey WC (2001) A multi-center controlled study of rapid hepatitis B vaccination using a novel triple antigen recombinant vaccine. Vaccine 19: 3437 - 3443.

## Claims

1. An expression cassette containing a sequence encoding the small subunit of the HBV surface antigen (S-HBsAg) and regulatory sequences controlling its expression: the 35S RNA promoter of the cauliflower mosaic virus (CaMV) and the nopaline synthase terminator (NOSt) sequence, wherein said expression cassette possesses the sequence designated as SEQ ID No. 1.

2. A T-DNA molecule containing flanking T-DNA sequences as well as two expression cassettes located between them:
- an expression cassette according to claim 1 and
- an expression cassette composed of a sequence encoding a herbicide resistance gene, preferentially the *bar* gene, as well as regulatory sequences controlling its expression, preferentially the nopaline synthase promoter (PNOS) and g7t transcription terminator.

3. An expression vector containing an expression cassette according to claim 1, preferentially a T-DNA molecule according to claim 2.

4. An *E. coli* cell containing an expression vector according to claim 3.

5. An *Agrobacterium tumefaciens* cell containing an expression vector according to claim 3.

6. A use of a plant expression vector according to claim 3 in the production of transgenic plants, preferentially lettuce.

7. A transgenic plant cell containing a plant expression vector according to claim 3 capable of expressing the HBV small surface antigen protein, S-HBsAg, and possessing resistance to phosphinothricine herbicides.

8. A cell according to claim 7, **characterised in that** it is a lettuce cell.

9. A use of a plant cell according to claims 7-8 in the production of an oral vaccine against viral hepatitis type B.

10. A use according to claim 9, **characterised in that** the plant cells used are in the form of plant biomass, particularly lyophilised plant material, preferentially lettuce.

11. A use according to claim 9, **characterised in that** the vaccine produced is in a form selected from among: a suspension, syrup, granulate, tablets or capsules.

## Patentansprüche

1. Expressionskassette, die eine Sequenz enthält, die die kleine Untereinheit des HBV-Oberflächenantigens (S-HBsAg) codiert, sowie regulatorische Sequenzen, die seine Expression steuern: den 35S-RNA-Promotor des Blumenkohl-Mosaik-Virus (Cauliflower Mosaic Virus/CaMV) und die Nopalinsynthaseterminator (NOSt)-Sequenz, wobei die Expressionskassette die als SEQ ID NO:1 bezeichnete Sequenz aufweist.

2. T-DNA-Molekül, das flankierende T-DNA-Sequenzen sowie zwei zwischen ihnen lokalisierte Expressionskassetten enthält:
- eine Expressionskassette nach Anspruch 1 und
- eine Expressionskassette, die aus einer Sequenz besteht, die ein Herbizidresistenz-Gen, bevorzugt das *ba*r-Gen, codiert, sowie aus regulatorischen Sequenzen, die seine Expression steuern, bevorzugt den Nopalinsynthasepromotor (PNOS) und den g7t-Transkriptionsterminator.

3. Expressionsvektor, der eine Expressionskassette nach Anspruch 1 enthält, bevorzugt ein T-DNA-Molekül nach Anspruch 2.

4. *E. coli*-Zelle, die einen Expressionsvektor nach Anspruch 3 enthält.

5. *Agrobacterium tumefaciens*-Zelle, die einen Expressionsvektor nach Anspruch 3 enthält.

6. Verwendung eines Pflanzenexpressionsvektors nach Anspruch 3 bei der Herstellung von transgenen Pflanzen, bevorzugt Salat.

7. Transgene Pflanzenzelle, die einen Pflanzenexpressionsvektor nach Anspruch 3 enthält, der in der Lage ist, das kleine HBV-Oberflächenantigen S-HBsAg zu exprimieren, und Resistenz gegenüber Phosphinothricinherbiziden aufweist.

8. Zelle nach Anspruch 7, die **dadurch gekennzeichnet ist, dass** es eine Salatzelle ist.

9. Verwendung einer Pflanzenzelle nach den Ansprüchen 7 bis 8 bei der Herstellung eines oralen Impfstoffs gegen Virushepatitis Typ B.

10. Verwendung nach Anspruch 9, **dadurch gekennzeichnet, dass** die verwendeten Pflanzenzellen in Form von Pflanzenbiomasse vorliegen, insbesondere in Form von lyophilisiertem Pflanzenmaterial, vorzugsweise Salat.

11. Verwendung nach Anspruch 9, **dadurch gekennzeichnet, dass** der hergestellte Impfstoff in einer Form vorliegt, die ausgewählt ist aus: einer Suspension, einem Sirup, einem Granulat, Tabletten oder Kapseln.

## Revendications

1. Cassette d'expression contenant une séquence codant pour la petite sous-unité de l'antigène de surface du VHB (S-HBsAg) et des séquences régulatrices régulant son expression : le promoteur d'ARN 35S du virus de la mosaïque du chou-fleur (CaMV) et le terminateur de la nopaline synthase (NOSt), ladite cassette d'expression possédant la séquence dénommée SEQ ID N° 1.

2. Molécule d'ADN-T contenant des séquences d'ADN-T encadrantes ainsi que deux cassettes d'expression situées entre elles :
- une cassette d'expression selon la revendication 1 et
- une cassette d'expression composée d'une séquence codant pour un gène de résistance aux herbicides, de préférence le gène *bar*, ainsi que des séquences régulatrices régulant son expression, de préférence le promoteur de la nopaline synthase (PNOS) et le terminateur de transcription g7t.

3. Vecteur d'expression contenant une cassette d'expression selon la revendication 1, de préférence une molécule d'ADN-T selon la revendication 2.

4. Cellule de *E. coli* contenant un vecteur d'expression selon la revendication 3.

5. Cellule d'*Agrobacterium tumefaciens* contenant un vecteur d'expression selon la revendication 3.

6. Utilisation d'un vecteur d'expression végétal selon la revendication 3 dans la production de plantes, de préférence de laitue, transgéniques.

7. Cellule végétale transgénique contenant un vecteur d'expression végétal selon la revendication 3 ayant la capacité d'exprimer la protéine majeure antigène de surface du VHB, S-HBsAg, et possédant une résistance aux herbicides à base de phosphinotricine.

8. Cellule selon la revendication 7, **caractérisée en ce qu'**il s'agit d'une cellule de laitue.

9. Utilisation d'une cellule végétale selon les revendications 7 et 8 dans la production d'un vaccin oral contre l'hépatite virale B.

10. Utilisation selon la revendication 9, **caractérisée en ce que** les cellules végétales utilisées se présentent sous la forme de biomasse végétale, en particulier de matériel végétal, de préférence de laitue, lyophilisé.

11. Utilisation selon la revendication 9, **caractérisée en ce que** le vaccin produit se présente sous une forme sélectionnée parmi : une suspension, un sirop, des granulés, des comprimés ou des capsules.


### Sequence listing

<110> Instytut Genetyki Roslin Polskiej Akademii Nauk et al
<120> Expression cassette, a T-DNA molecule, plant expression vector
   , transgenic plant cell as well as their use in the production of a
vaccine
<130> PZ/0407/RW/PCT
<160> 2
<170> PatentIn version 3.3
<210> 1
   <211> 2252
   <212> DNA
   <213> Artificial
<220>
   <223> Sequence of the expression cassette P35S-SHBs-NOSt placed in the
   binary vector pKHBSBAR
<400> 1
   gaattcccca gattagcctt ttcaatttca gaaagaatgc taacccacag atggttagag      60
   aggcttacgc agcaggtctc atcaagacga tctacccgag caataatctc caggaaatca     120
   aataccttcc caagaaggtt aaagatgcag tcaaaagatt caggactaac tgcatcaaga     180
   acacagagaa agatatattt ctcaagatca gaagtactat tccagtatgg acgattcaag     240
   gcttgcttca caaaccaagg caagtaatag agattggagt ctctaaaaag gtagttccca     300
   ctgaatcaaa ggccatggag tcaaagattc aaatagagga cctaacagaa ctcgccgtaa     360
   agactggcga acagttcata cagagtctct tacgactcaa tgacaagaag aaaatcttcg     420
   tcaacatggt ggagcacgac acacttgtct actccaaaaa tatcaaagat acagtctcag     480
   aagaccaaag ggcaattgag acttttcaac aaagggtaat atccggaaac ctcctcggat     540
   tccattgccc agctatctgt cactttattg tgaagatagt ggaaaaggaa ggtggctcct     600
   acaaatgcca tcattgcgat aaaggaaagg ccatcgttga agatgcctct gccgacagtg     660
   gtcccaaaga tggaccccca cccacgagga gcatcgtgga aaaagaagac gttccaacca     720
   cgtcttcaaa gcaagtggat tgatgtgata tctccactga cgtaagggat gacgcacaat     780
   cccactatcc ttcgcaagac ccttcctcta tataaggaag ttcatttcat ttggagagaa     840
   cacgggggac tctagaggat ccatggagaa catcacatca ggattcctag gacccctgct     900
   cgtgttacag gcggggtttt tcttgttgac aagaatcctc acaataccgc agagtctaga     960
   ctcgtggtgg acttctctca attttctagg gggaactacc gtgtgtcttg gccaaaattc    1020
   gcagtcccca acctccaatc actcaccaac ctcctgtcct ccaacttgtc ctggttatcg    1080
   ctggatgtgt ctgcggcgtt ttatcatctt cctcttcatc ctgctgctat gcctcatctt    1140
   cttgttggtt cttctggact atcaaggtat gttgcccgtc tgtcctctaa ttccaggatc    1200
   ttcaacaacc agcgtgggac catgcagaac ctgcacgact actgttcaag gaacctctat    1260
   gtatccctcc tgttgctgta ccaaaccttc ggacggaaat tgcacctgta ttcccatccc    1320
   atcatcctgg gctttcggaa aattcctatg ggagtgggcc tcagcccgtt tctcctggct    1380
   cagtttacta gtgccatttg ttcagtggtt cgtagggctt tcccccactg tttggctttc    1440
   agttatatgg atgatgtggt attgggggcc aagtctgtac agcatcttga gtcccttttt    1500
   accgctgtta ccaattttct tttgtctttg ggtatacatt taactgcagc tcgagtaaag    1560
   aaggagtgcg tcgaagcaga tcgttcaaac atttggcaat aaagtttctc aagattgaat    1620
   cctgttgccg gtcttgcgat gattatcata taatttctgt tgaattacgt taagcatgta    1680
   ataattaaca tgtaatgcat gacgttattt atgagatggg tttttatgat tagagtcccg    1740
   caattataca tttaatacgc gatagaagac aaaatatagc gcgcaaacta ggataaatta    1800
   tcgcgcgcgg tgtcatctat gttactagat cgatcaaact tcggcactgt gtaatgacga    1860
   tgagcaatcg agaggctgac taacaaaagg tatgcccaaa aacaacctct ccaaactgtt    1920
   tcgaattgga agtttctgct catgccgaca ggcataactt agatattcgc gggctattcc    1980
   cactaattcg tcctgctggt ttgcgccaag ataaatcagt gcatctcctt acaagttcct    2040
   ctgtcttgtg aaatgaactg ctgactgccc cccaagaaag cctcctcatc tcccagttgg    2100
   cggcggctga tacaccatcg aaaacccacg tccgaacact tgatacatgt gcctgagaaa    2160
   taggcctacc tcaagagcaa gtcctttctg tgctcgtcgg aaattcctct cctgtcagac    2220
   ggtcgtgcgc atgtcttgcg ttgatgaagc tt                                  2252
<210> 2
   <211> 1124
   <212> DNA
   <213> Artificial
<220>
   <223> Sequence of the expression cassette PNOS-bar-g7t placed in the
   binary vector pKHBSBAR
<400> 2
   aagcttaaca ctgatagttt aaactgaagg cgggaaacga caatctgatc atgagcggag     60
   aattaaggga gtcacgttat gacccccgcc gatgacgcgg gacaagccgt tttacgtttg    120
   gaactgacag aaccgcaacg ttgaaggagc cactcagccg cgggtttctg gagtttaatg    180
   agctaagcac atacgtcaga aaccattatt gcgcgttcaa aagtcgccta aggtcactat    240
   cagctagcaa atatttcttg tcaaaaatgc tccactgacg ttccataaat tcccctcggt    300
   atccaattag agtctcatat tcactctcaa tccaaataat ctgcaagatc tatgagccca    360
   gaacgacgcc cggccgacat ccgccgtgcc accgaggcgg acatgccggc ggtctgcacc    420
   atcgtcaacc actacatcga gacaagcacg gtcaacttcc gtaccgagcc gcaggaaccg    480
   caggagtgga cggacgacct cgtccgtctg cgggagcgct atccctggct cgtcgccgag    540
   gtggacggcg aggtcgccgg catcgcctac gcgggcccct ggaaggcacg caacgcctac    600
   gactggacgg ccgagtcgac cgtgtacgtc tccccccgcc accagcggac gggactgggc    660
   tccacgctct acacccacct gctgaagtcc ctggaggcac agggcttcaa gagcgtggtc    720
   gctgtcatcg ggctgcccaa cgacccgagc gtgcgcatgc acgaggcgct cggatatgcc    780
   ccccgcggca tgctgcgggc ggccggcttc aagcacggga actggcatga cgtgggtttc    840
   tggcagctgg acttcagcct gccggtaccg ccccgtccgg tcctgcccgt caccgagatc    900
   tgatgacccc tagaggatcc atcttgaaag aaatatagtt taaatattta ttgataaaat    960
   aacaagtcag gtattatagt ccaagcaaaa acataaattt attgatgcaa gtttaaattc   1020
   agaaatattt caataactga ttatatcagc tggtacattg ccgtagatga aagactgagt   1080
   gcgatattat gtgtaataca taaattgatg atatagctag ctta                   1124